(19) Europäisches Patentamt
European Patent Office
Office européen des brevets

(11) Veröffentlichungsnummer : **0 081 674**
**B1**

(12) # EUROPÄISCHE PATENTSCHRIFT

(45) Veröffentlichungstag der Patentschrift : **08.07.87**

(51) Int. Cl.[4] : **C 07 D277/46**, C 07 D501/20, C 07 D501/34, C 07 D501/36

(21) Anmeldenummer : **82110254.8**

(22) Anmeldetag : **06.11.82**

(54) **Zwischenprodukte, Verfahren zu deren Herstellung und Verfahren zur Herstellung von Cephalosporinen.**

(30) Priorität : **19.11.81 DE 3145727**

(43) Veröffentlichungstag der Anmeldung : **22.06.83 Patentblatt 83/25**

(45) Bekanntmachung des Hinweises auf die Patenterteilung : **08.07.87 Patentblatt 87/28**

(84) Benannte Vertragsstaaten : **AT BE CH DE FR GB IT LI LU NL SE**

(56) Entgegenhaltungen :
**EP-A- 0 049 448**
**Patent Abstracts of Japan, Band 5, Nr.41, 18.März 1981**
**Patent Abstracts of Japan, Band 6, Nr.3, 9.Jan.1983**

(73) Patentinhaber : **BAYER AG**
**Konzernverwaltung RP Patentabteilung**
**D-5090 Leverkusen 1 Bayerwerk (DE)**

(72) Erfinder : **Kinast, Günther, Dr.**
**Am Eckbusch 35/33**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Cephalosporine der allgemeinen Formel I sind in der älteren deutschen Patentanmeldung P 30 37 997 6 genannt. Diese Verbindungen haben eine breite antibakterielle Wirkung sowohl gegen gramnegative wie auch gegen grampositive Bakterien.

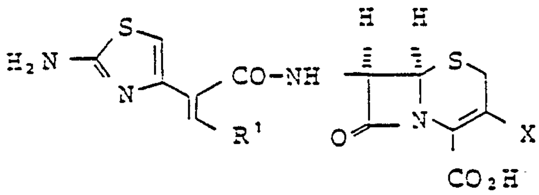

(I)

$R^1$ = Alkyl, Aryl

Nach dem in dieser älteren Anmeldung genannten Verfahren werden die Verbindungen der Formel I nach dem in dem folgenden Formelschema aufgeführten Verfahren hergestellt :

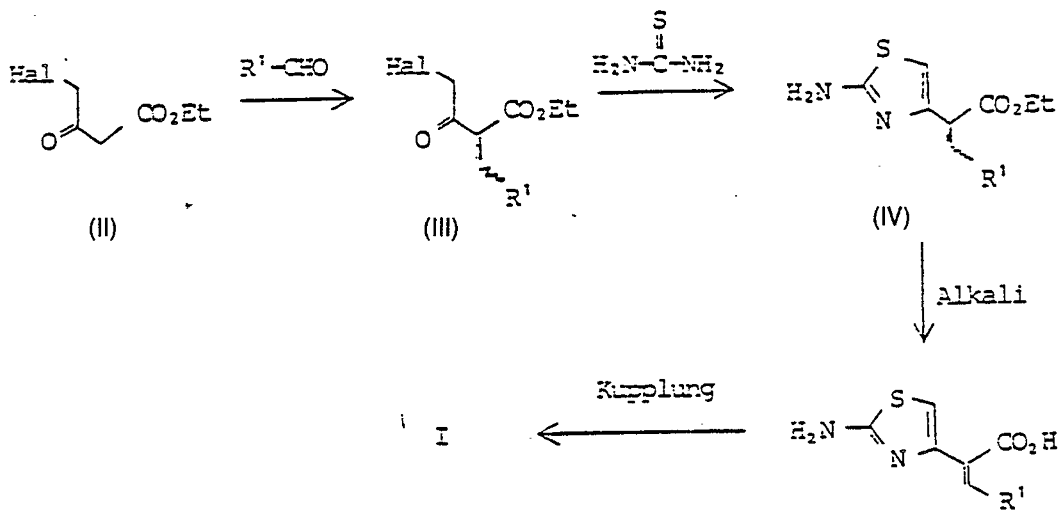


(II) (III) (IV) (V)

Dieses Verfahren führt jedoch für Verbindungen der Formel I mit $R^1$ = Alkyl nur zu unbefriedigenden Ausbeuten. So erhält man — z. B. für den Fall $R^1$ = Isopropyl — bei der Umsetzung von III mit Thioharnstoff durch Dekonjugation der Doppelbindung und durch Michaeladdition neben der gewünschten Verbindung IV die Produkte der Formel VI und VII zum bei weitem größten Anteil.

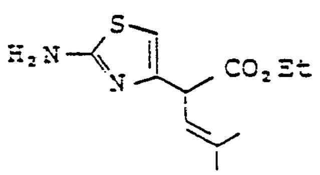

(VI)

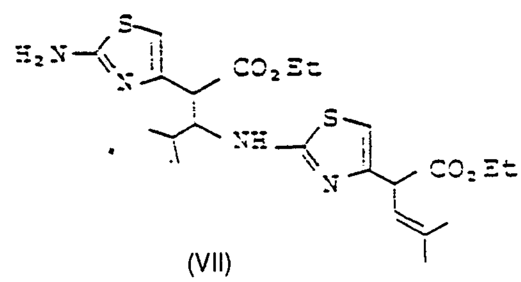

(VII)

Weiterhin tritt für $R^1$ = Alkyl bei der Kupplung der Säuren V an die 7-Aminocephalosporansäuren zu den Produkten der Formel I zu einem großem Teil eine Isomerisierung der Doppelbindung zu den Produkten der Formel VIII ein, wenn man nach den in der genannten Anmeldung beschriebenen Verfahren (z. B. Hydroxybenztriazol/DCC) arbeitet. Die Verbindungen der Formel VIII aber

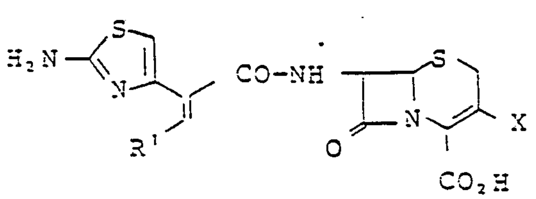

(VIII)

zeigen im allgemeinen nur etwa 1/10 der biologischen Aktivität der Verbindungen der Formel I.

Es wurde nun ein Verfahren zur Herstellung der Verbindungen der Formel I, das über neue Zwischenprodukte verläuft, gefunden und das die oben aufgeführten Nachteile nicht hat. Dieses neue Verfahren ist sowohl für $R^1$ = Alkyl als auch für $R^1$ = Aryl anwendbar und ist im folgenden Reaktionsschema zusammengefaßt :

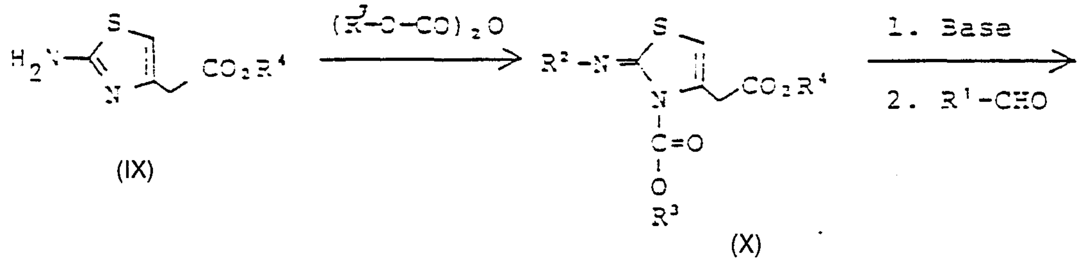


(IX) (X)

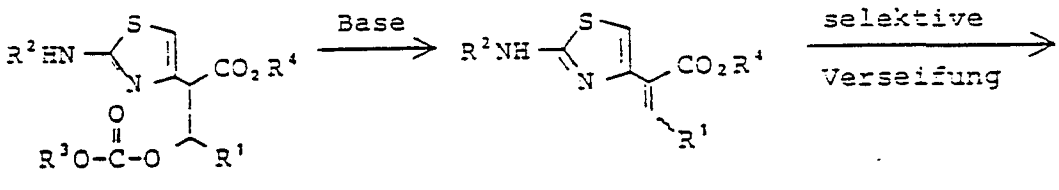


(XI) (XII)

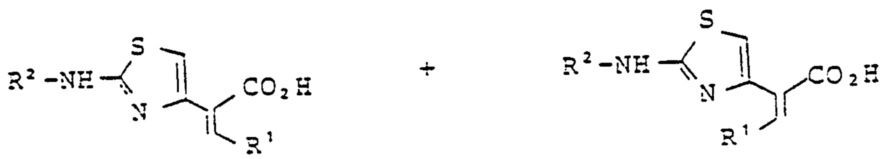

(XIII) (XIV)

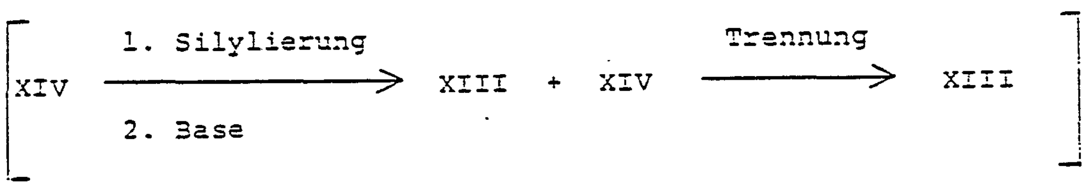


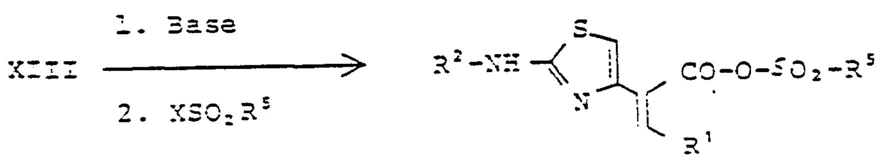


(XV) (XVI)

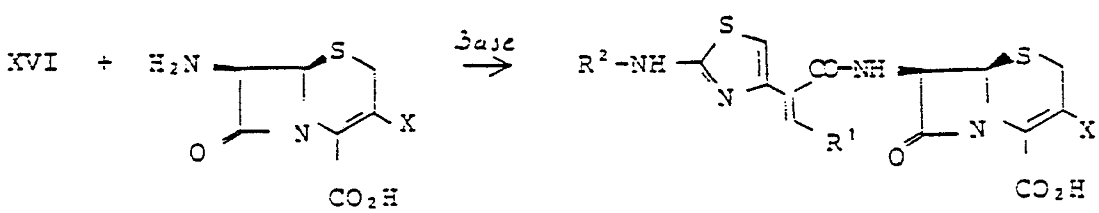


(XVII) (XVIII)

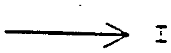

Als erstes werden die Verbindungen der Formel X hergestellt.

Die Verbindungen der Formel X sind neu und daher sind die Verbindungen der Formel X

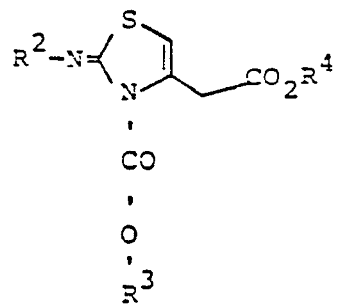

(X)

in der

$R^2$ $CO_2R^3$

$R^3$ und $R^4$ gleich oder verschieden sein können und ein gegebenenfalls substituierter Alkylrest mit 1-15 C-Atomen, ein gegebenenfalls substituierter Alkenylrest mit 3-15 C-Atomen, ein gegebenenfalls substituierter Cycloalkylrest mit 3-10 C-Atomen, ein gegebenenfalls substituierter Cycloalkenylrest mit 5-10 C-Atomen, ein gegebenenfalls substituierter Arylrest mit 1 bis 3 Ringen oder ein gegebenenfalls substituierter Heterocyclylrest mit 1-3 Ringen, die bis zu 5 Stickstoff-, Schwefel- oder Sauerstoffatome enthalten können, sind und die genannten Alkyl-, Alkenyl-, Cycloalkyl- und Cycloalkenylreste durch Alkylreste mit 1-4 C-Atomen, O-Alkylreste mit 1-4 C-Atomen, Halogen, gegebenenfalls substituierte Phenylreste, $C \equiv N$ und $C_1$-$C_5$-Trialkylsilyl substituiert sein können und die Aryl- und Heterocyclylreste einschließlich der genannten Phenylreste durch Alkyl-, O-Alkyl-, S-Alkyl-, Alkyloxycarbonyl-, Halogen- und Phenylreste, sowie durch Nitro und $C \equiv N$ substituiert sein können, wobei alle Alkylreste 1 bis 4 C-Atome haben, Gegenstand der Erfindung.

Wenn die Reste $R^3$ und/oder $R^4$ substituiert sind, so können sie 1-5, vorzugsweise 1 oder 2 Substituenten tragen.

Besonders vorteilhaft für das Verfahren ist es, wenn

$R^2$ = eine basenstabile, im Sauren abspaltbare Schutzgruppe wie z. B. tert.-Butoxycarbonyl ist, und wenn

$R^4$ = ein basich verseifbarer Rest wie z. B. Methyl oder Ethyl ist.

Die Verbindungen der Formel X erhält man, indem man die an sich bekannten Verbindungen der Formel IX (siehe z. B. E. Campaine und T.P. Selby, J. Heterocycl. Chem. 17 (1980)), in einem geeigneten Lösungsmittel mit einem Pyrokohlensäureester der Formel $R^3$—O—CO—O—CO—O—$R^3$ reagieren läßt.

Weiterer Gegenstand der Erfindung ist daher ein Verfahren zur Herstellung der Verbindungen der Formel X

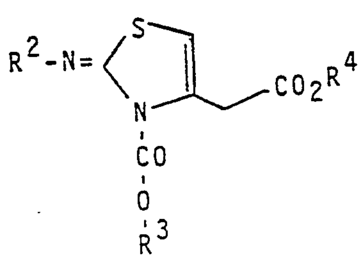

(X)

in denen

$R^2$ $CO_2R^3$

$R^3$ und $R^4$ gleich oder verschieden sein können und ein gegebenenfalls substituierter Alkylrest mit 1-15 C-Atomen, ein gegebenenfalls substituierter Alkenylrest mit 3-15 C-Atomen, ein gegebenenfalls substituierter Cycloalkylrest mit 3-10 C-Atomen, ein gegebenenfalls substituierter Cycloalkenylrest mit 5-10 C-Atomen, ein gegebenenfalls substituierter Arylrest mit 1-3 Ringen oder ein gegebenenfalls substituierter Heterocyclylrest mit 1-3 Ringen, die bis zu 5 Stickstoff-, Schwefel- oder Sauerstoffatome enthalten können, sind und die genannten Alkyl-, Alkenyl-, Cycloalkyl- und Cycloalkenylreste durch Alkylreste mit 1-4 C-Atomen, O-Alkylreste mit 1-4 C-Atomen, Halogen, gegebenenfalls substituierte Phenylreste, $C \equiv N$ und $C_1$-$C_5$-Trialkylsilyl substituiert sein können und die Aryl- und Heterocyclylreste einschließlich der genannten Phenylreste durch Alkyl-, O-Alkyl-, S-Alkyl-, Alkyloxycarbonyl-, Halogen- und Phenylreste, sowie durch Nitro und $C \equiv N$ substituiert sein können, wobei alle Alkylreste 1 bis 4 C-Atome haben, das dadurch gekennzeichnet ist, daß man eine Verbindung der Formel IX

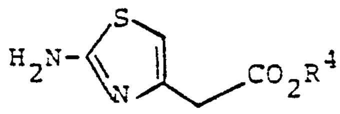

(IX)

mit einem Pyrokohlensäureester der Formel

$R^3$—O—CO—O—CO—O—$R^3$

in einem geeigneten Lösungsmittel umsetzt, wobei $R^3$ und $R^4$ die obengenannte Bedeutung haben.

Als Lösungsmittel eignen sich besonders aprotische, polare Lösungsmittel wie z. B. Acetonitril, Dimethylformamid, Hexamethylphosphorsäuretriamid oder Dimethylsulfoxid, besonders die beiden letzten. Die Reaktion läuft besonders vorteilhaft bei Zimmertemperatur oder bei niedrigeren Temperaturen z. B. 0 bis — 50 °C ab, wobei man die Komponenten 1-7 Tage miteinander reagieren läßt. Der Pyrokohlensäureester wird im allgemeinen mit 2-2,5 Moläquivalenten eingesetzt.

Andere Lösungsmittel, höhere Temperaturen oder Acylierungskatalysatoren wie 4-Dimethylaminopyridin begünstigen stark die Bildung der unerwünschten Produkte der Formel XIX.

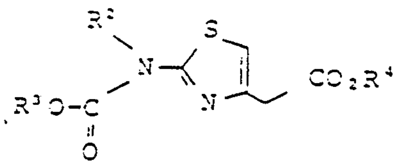

(XIX)

Zur Herstellung der neuartigen Verbindungen der Formel XI versetzt man die Verbindungen der Formel X in einem geeigneten Lösungsmittel bei tiefen Temperaturen mit 1 bis 1,1 Äquivalenten einer Base und gibt anschließend 1 bis 1,2 Äquivalente eines Aldehyds der Formel $R^1$—CHO hinzu.

Als Lösungsmittel für die Reaktion können z. B. Dimethylformamid, Dimethylsulfoxid, Diethylether, Tetrahydrofuran oder Toluol — bevorzugt Tetrahydrofuran — und als Base Alkoholate, Hydride, Amide oder Metallorganyle — bevorzugt Kalium-tert.-Butylat, Lithium-diisopropylamid und Buthyllithium — verwendet werden. Zur Durchführung der Reaktion gibt man die Base bei — 50 bis — 80 °C zu einer Lösung von X und fügt anschließend bei — 50 bis — 60 °C den Aldehyd hinzu und rührt ca. 12 Stunden bei — 50 bis — 60 °C. Zur Isolierung der Produkte der Formel XI wird der Ansatz neutralisiert und aufgearbeitet.

Ein weiterer Gegenstand der Erfindung sind auch die Verbindungen der Formel XI

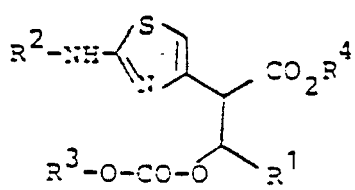

(XI)

in der $R^2$, $R^3$ und $R^4$ die oben für Verbindungen der Formel X angegebene Bedeutung haben und

$R^1$ einen gegebenenfalls substituierten Alkylrest mit 1-15 C-Atomen, einen gegebenenfalls substituierten Cycloalkylrest mit 3-10 C-Atomen, einen gegebenenfalls substituierten Arylrest mit 1 bis 2 Ringen oder einen gegebenenfalls substituierten Heterocyclylrest mit 1 bis 3 Ringen, die bis zu 5 Stickstoff-, Schwefel- und Sauerstoffatome enthalten können,

darstellt, und die genannten Alkyl- und Cycloalkylreste durch Alkylreste mit 1-6 C-Atomen, O-Alkylreste mit 1-6 C-Atomen, S-Alkylreste mit 1-6 C-Atomen, N-Alkylreste mit 1-6 C-Atomen, Alkyloxycarbonylreste mit 1-6 C-Atomen und gegebenenfalls substituierte Phenylreste substituiert sein können und die Aryl- und Heterocyclylreste einschließlich der genannten Phenylreste durch Alkyl-, O-Alkyl-, S-Alkyl-, Alkyloxycarbonyl-, Halogen-, vorzugsweise Chlor, und Phenylreste, wobei alle Alkylreste 1-6 C-Atome tragen, substituiert sein können.

Wenn $R^1$ einen durch die obengenannten Substituenten substituierten Rest darstellt, so sind 1-5, vorzugsweise 1-2 Substituenten bevorzugt.

Ganz besonders bedeutet

$R^1$ einen Alkylrest mit 1-10 C-Atomen und einen Cycloalkylrest mit 3-10 C-Atomen, die jeweils durch 1-2 Alkylreste mit 1-6 C-Atomen und/oder 1-2 Phenylreste substituiert sein können.

Zur Durchführung des Verfahrens zur Herstellung der Verbindungen der Formel I ist es nicht notwendig, die Verbindungen der Formel XI zu isolieren. Es ist vielmehr zweckmäßig, sie in situ direkt in die Verbindungen der Formel XII umzuwandeln. Hierzu reicht es im allgemeinen aus, den Ansatz nach Zugabe des Aldehyds $R^1$—CHO auf Raumtemperatur erwärmen zu lassen und über Nacht bei Raumtemperatur zu rühren. Sollte bis dahin die Eliminierung von XI nach XII nicht vollständig sein, gibt

man 1 bis 1,2 Äquivalente einer Base — wie z. B. ein Hydrid, ein Alkoholat oder ein Amid — besonders Kalim-tert.-butylat hinzu und rührt etwa 10 h bei Raumtemperatur.

Hatte man dagegen die Verbindung der Formel XI zuvor isoliert, so gibt man zur Herstellung der Verbindungen der Formel XII zu einer Lösung der Verbindungen der Formel XI in einem geeigneten Lösungsmittel 1.1 bis 2.2 Äquivalente einer Base. Als Lösungsmittel und als Base können die bei der Umsetzung von X nach XI genannten Verwendung finden, bevorzugt Tetrahydrofuran und Kalium-tert.-butylat.

Man erhält die Verbindungen der Formel XII als E/Z-Isomerengemische, die sich z. B. durch Umkristallisieren oder durch Säulenchromatographie an Kieselgel trennen lassen.

In den Verbindungen der Formel XII haben $R^1$, $R^2$ und $R^4$ die gleiche Bedeutung wie in den Verbindungen der Formel XI.

Zur Herstellung der Z-Carbonsäuren der Formel XIII kann man die Z-Ester, die man durch Trennung des E/Z-Isomerengemische der Ester der Formel XII erhalten kann, verseifen. Es ist aber für die Durchführung des Verfahrens zur Herstellung der Verbindungen der Formel I günstiger, das E/Z-Isomerengemisch der Ester der Formel XII in der Weise selektiv zu verseifen, daß man unter milden Bedingungen erst die E-Ester in die E-Carbonsäuren der Formel XIV überführt und abtrennt und anschließend die verbleibenden Z-Ester, in denen die Estergruppe sterisch stärker abgeschirmt ist, unter drastischeren Bedingungen zu den Z-Carbonsäuren der Formel XIII verseift.

Die milden Verseifungsbedingungen, die zu den E-Carbonsäuren XIV führen, sind z. B. Ethanol/2 N Natronlauge/Raumtemperatur/24 h. Zweckmäßigerweise führt man die Verseifung in der Art durch, daß man nach der Überführung der Verbindungen der Formel XI in die Verbindungen der Formel XII direkt zu dem Reaktionsansatz 2 N Natronlauge gibt, und bei Raumtemperatur oder unter leichtem Erwärmen so lange rührt, bis die E-Ester verseift sind. Dann trennt man durch Extraktion im Alkalischen die Z-Ester von dem Ansatz ab und verseift sie unter drastischeren Bedingungen.

Drastischere Verseifungsbedingungen sind z. B. Ethanol/2 N Natronlauge/24 h Rückfluß — eventuell auch stärkere Natronlauge oder höherer siedende Lösungsmittel wie z. B. Dioxan.

Auf diese Weise erhält man die gewünschten Z-Carbonsäuren der Formel XIII und die E-Carbonsäuren der Formel XIV. Letztere lassen sich nach Überführung in die Silylester — z. B. mit Bistrimethylsilylacetamid — in einem geeigneten Lösungsmittel — z. B. Diethylether oder Tetrahydrofuran — mit einer Base wie Kalium-tert.-butylat und anschließender Hydrolyse mit verdünnter Säure wieder in ein Gemisch der E-Carbonsäuren der Formel XIV und der Z-Carbonsäuren der Formel XIII überführen.

Aus diesem E/Z-Isomerengemisch lassen sich die Z-Carbonsäuren der Formel XIII z. B. durch Kristallisation oder durch Trennung an einem Ionenaustauscher rein isolieren. Die Trennung mit Hilfe von Ionenaustauschern ist einfach, da die Z-Carbonsäuren der Formel XIII sehr viel stärker sauer sind als die E-Carbonsäuren der Formel XIV. So werden die E-Carbonsäuren der Formel XIV bereits mit Methanol von schwach-basischen Ionenaustauschern eluiert, die Z-Carbonsäuren der Formel XIII dagegen erst nach Zusatz von Elektrolyten, z. B. 2 N Natronlauge. Unter schwach basischen Ionenaustauschern sind solche Ionenaustauscher in fester oder flüssiger Form zu verstehen, die tertiäre Aminogruppen enthalten wie z. B. Lewatit MP 62.

In den Verbindungen der Formel XIII und XIV haben $R^1$ und $R^2$ die gleiche Bedeutung wie bei den Verbindungen der Formel XII. Zusätzlich kann $R^2 = H$ sein, wenn vor der Verseifung in den Verbindungen der Formel XII $R^2$ eine alkalisch verseifbare Schutzgruppe wie z. B. Methyloxycarbonyl war. Für die Durchführung des Verfahrens zur Herstellung der Verbindungen der Formel I ist es aber zweckmäßiger, wenn $R^2$ eine Schutzgruppe ist, die unter den Verseifungsbedingungen stabil ist — bevorzugt tert.-Butyloxycarbonyl.

Für die Kupplung von Carbonsäuren an 7-Aminocephalosporansäuren sind in der Cephalosporinchemie eine große Anzahl von Methoden bekannt, die sich letzlich aus der Peptidchemie ableiten. Bei den Versuchen zur Knüpfung der Amidbindung zwischen den Z-Carbonsäuren der Formel XIII und den Cephalosporansäuren der Formel XVII versagen diese Methoden jedoch oder führen nur zu sehr schlechten Ausbeuten, insbesondere dann, wenn $R^1$ ein Alkylrest ist. Die Gründe hierfür sind in der großen sterischen Hinderung der Carboxylgruppe in den Carbonsäuren der Formel XIII durch den Rest $R^1$ sowie in der ausgeprägten Neigung des Restes $R^1$, nach Aktivierung der Carboxylfunktion — z. B. Überführung in das Säurechlorid — in die E-Form zu isomerisieren. Man erhält dann nach Reaktion mit den 7-Aminocephalosporansäuren der Formel XVII nicht die gewünschten Verbindungen der Formel XVIII, sondern die Verbindungen der Formel XXI oder Gemische aus beiden.

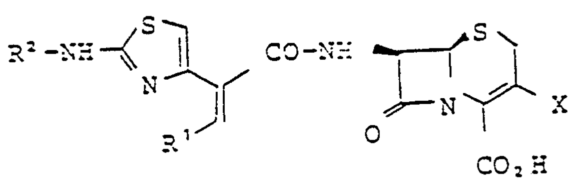

(XXI)

Es wurde nun gefunden, daß man die Z-Carbonsäuren der Formel XIII ohne die oben aufgeführten Nachteile auf einfache, schonende und billige Weise aktivieren kann, wenn man sie bei tiefen Temperaturen in die gemischten Anhydride der Formel XVI überführt.

Diese Verbindungen der Formel XVI sind neu und ebenfalls Gegenstand der Erfindung. In diesen Verbindungen haben $R^1$ und $R^2$ dieselbe Bedeutung wie in den Verbindungen der Formel XII.

$R^5$ bedeutet F, einen gegebenenfalls substituierten Alkyl-, Alkenyl-, Cycloalkyl-, Cycloalkenyl-, Aryl- oder Heterocyclylrest, wobei Halogen-, Alkyl-, Aryl-, O-Alkyl-, S-Alkyl-, CN-, Alkoxycarbonyl-, Nitrogruppen u. a. als Substituenten auftreten können.

Im Prinzip kann $R^5$ jeder beliebige, organische Rest sein, von dem ein Säurechlorid, Bromid oder Anhydrid der Formel XV bekannt oder herstellbar ist,

$$ZSO_2R^5 \qquad Z = Br, Cl, OSO_2R^5. \qquad (XV)$$

Besonders ist

$R^5$ ein Alkylrest mit 1-10 C-Atomen, der gegebenenfalls durch Fluor, Chlor, CN, Phenyl, Alkyloxycarbonyl, Alkyloxy oder Alkyl substituiert sein kann, wobei letztere Alkylreste 1-4 C-Atome tragen können, und ein Phenylrest, der gegebenenfalls durch Fluor, Chlor, Brom, CN, Alkyl, Alkyloxy, Alkylthio, Alkyloxycarbonyl — wobei letztere Alkylgruppen 1-4 C-Atome tragen können — Nitro, Trifluormethyl und Phenyl substituiert sein kann.

Wenn $R^5$ substituiert ist, sind vorzugsweise 1-3 Substituenten, vorzugsweise die genannten vorhanden.

Ganz besonders bevorzugt stellt $R^5$ einen Methyl- oder p-Tolylrest dar.

Solche gemischten Anhydride der Formel XVI können hergestellt werden, indem man die Carbonsäure XIII und ein geeignetes Amin in äquimolaren Mengen in einem geeigneten Lösungsmittel löst und mit 1 bis 1.05 Äquivalenten eines Sulfonsäurederivates der Formel XV reagieren läßt.

Als Lösungsmittel eignen sich alle Solventien, die unter den Reaktionsbedingungen stabil sind, wie z. B. Diethylester, Tetrahydrofuran, Acetonitril, Aceton, Methylenchlorid, Chloroform oder Dimethylformamid.

Als Amin eignen sich tertiäre Amine wie z. B. Triethylamin oder Tributylamin aber auch sterisch gehinderte sekundäre Amine wie z. B. Diisopropylamin.

Die Umsetzungen können bei Temperaturen zwischen — 80 °C und Raumtemperatur durchgeführt werden, wobei tiefe Temperaturen eine Isomerisierung der Substituenten an der Doppelbindung vermeiden. Vorteilhaft werden die Reaktionen bei — 20 bis — 50 °C bei einer Reaktionsdauer von 1 bis 10 h durchgeführt.

Die Verbindungen der Formel XVI können isoliert werden, indem man z. B. in Tetrahydrofuran als Lösungsmittel und mit Triethylamin als Base arbeitet, das gebildete Triethylaminhydrochlorid absaugt und das Lösungsmittel im Vakuum abdestilliert. Es ist aber zweckmäßiger, die erhaltenen Lösungen der Verbindungen der Formel XVI direkt mit den Cephalosporansäuren der Formel XVII umzusetzen. Hierzu löst man die Cephalosporansäuren der Formel XVII in einem geeigneten Lösungsmittel mit 2-4 Äquivalenten eines Amins, kühlt die Lösung auf die gewünschte, anschließende Reaktionstemperatur vor und gibt bei dieser Temperatur diese Lösung zu der oben beschriebenen Lösung der Verbindung der Formel XVI. Zur Vermeidung einer Isomerisierung des Restes $R^1$ in den Reaktionsprodukten der Formel I führt man die Reaktion zweckmäßigerweise bei — 60 bis — 30 °C durch und läßt den Ansatz über Nacht auf Raumtemperatur kommen.

Zum Lösen der Cephalosporansäuren der Formel XVII können die bei der Herstellung der Verbindungen der Formel XVI genannten Amine und Lösungsmittel verwendet werden. Sollte man auf diese Weise keine Lösungen mit befriedigenden Konzentrationen der Cephalosporansäuren der Formel XVII erhalten, kann man selbstverständlich auch die gut löslichen, aus der Cephalosporinchemie hinreichend bekannten Ester der Verbindungen der Formel XVII einsetzen, wie z. B. Silyl-, tert.-Butyl- oder Diphenylmethylester.

Nach Aufarbeitung erhält man die Verbindungen der Formel XVIII, in denen $R^1$ und $R^2$ die bei den Verbindungen der Formel XVI genannten Bedeutungen aufweisen und X eine bei Cephalosporinen übliche Gruppen darstellt, z. B. Wasserstoff, $C_1$-$C_4$-Alkyl, Halogen, $C_1$-$C_4$-Alkoxy, Hydroxymethyl, Formyloxymethyl, $C_1$-$C_4$-Alkylcarbonyloxymethyl, Aminocarbonyloxymethyl, Pyridiniummethyl, 4-Carbamoyl-pyridiniummethyl oder Heterocyclylthiomethyl bedeutet, wobei Heterocyclyl vorzugsweise für einen Rest der Formel

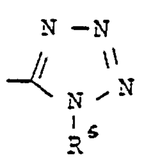 , 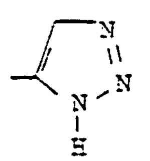 , 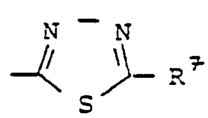 , 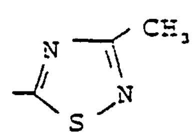

steht, worin

$R^6$ Wasserstoff, Methyl, 2-Dimethylaminoethyl, Carboxymethyl oder Sulfomethyl und

$R^7$ Wasserstoff oder Methyl bedeuten.

Bevorzugt bedeutet

X Wasserstoff, Chlor, Methoxy, Hydroxymethyl, Acetyloxymethyl, Aminocarbonyloximethyl, Pyridiniummethyl,

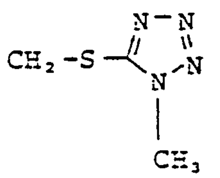 , 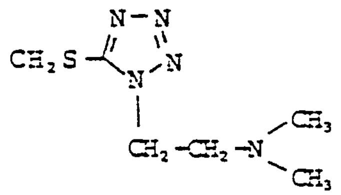 oder 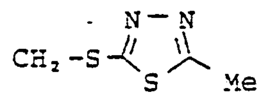

Aus den Verbindungen der Formel XVIII erhält man nach Abspaltung der Schutzgruppe $R^2$ die Verbindung der Formel I, in denen $R^1$ und X die bei den Verbindungen der Formel XVIII genannte Bedeutung aufweisen. Wie schon bei den Verbindungen der Formel X erwähnt, ist es für die gesamte Reaktionssequenz zur Herstellung der Verbindungen der Formel I aus den Verbindungen der Formel X äußerst vorteilhaft, wenn $R^2$ eine basenstabile Schutzgruppe ist, die sich selektiv abspalten läßt wie z. B. tert.-Butyloxycarbonyl (Abspaltung mit Trifluoressigsäure).

Beispiel 1

2-tert.-Butoxycarbonylimino-3-tert.-butoxycarbonyl-4-thiazolin-4-yl-essigsäureethylester

186 g (1 Mol) 2-Aminothiazol-4-yl-essigsäureethylester, 300 ml Dimethylsulfoxid und 500 g (2,3 Mol) 98 %iges Di-tert.-butyl-pyrocarbonat werden 7 d bei Raumtemperatur gerührt. Dann gibt man unter Eiskühlung bei max. 20 °C 3,5 l Eiswasser hinzu, rührt 30 min, saugt den Niederschlag ab, wäscht ihn mit 2 l Wasser und nimmt ihn in 2 l Methylenchlorid auf. Das Wasser wird abgetrennt, die Methylenchloridphase über $Na_2SO_4$ getrocknet und einrotiert. Das erhaltene Öl wird sofort (bevor die Kristallisation einsetzt) zur Kristallisation in 2 l Petrolether aufgenommen. Ausbeute 302 g (78 %), Fp. 90 °C.

Beispiel 2

2-tert.-Butoxycarbonylimino-3-tert.-butoxycarbonyl-4-thiozolin-4-yl-essigsäuremethylester

wird analog Beispiel 1 aus 2-Aminothiazol-4-yl-essigsäuremethylester hergestellt. Ausbeute 67 %, Fp. 67-69 °C.

Beispiel 3

2-Ethoxycarbonylimino-3-ethoxycarbonyl-4-thiazolin-4-yl-essigsäureethylester

wird analog Beispiel 1 aus 2-Aminothiazol-4-yl-essigsäureethylester und Diethylpyrocarbonat hergestellt. Ausbeute 71 %, Fp. 102 °C.

Beispiel 4

2-tert.-Butoxycarbonylimino-3-tert.-butoxycarbonyl-4-thiazolin-4-yl-essigsäure-tert.-butylester

157 g (0,5 Mol) 2-Aminothiazol-4-yl-essigsäure-tert.-butylester, 150 ml Dimethylsulfoxid und 260 g (1,2 Mol) 98 %iges Di-tert.-butylpyrocarbonat wurden analog Beispiel 1 umgesetzt. Ausbeute 62 %.

Beispiel 5

2-Aminothiazol-4-yl-essigsäure-trimethylsilylethylester

Zu 7,9 g (0,05 Mol) 2-Aminothiazol-4-yl-essigsäure in 50 ml Acetonitril gibt man bei Raumtemperatur 11,2 g (15,8 ml, 0,1 Mol) Trimethylsilylethanol, 100 mg 4-Dimethylaminopyridin und 11,4 g Dicyclohexylcarbodiimid und rührt 2 d. Dann wird der ausgefallene Harnstoff abgesaugt, mit Ether gewaschen, der Ablauf einrotiert, der Rückstand in Ether aufgenommen, die etherische Lösung mit 0,5 N Salzsäure und mit $NaHCO_3$-Lösung gewaschen, über $MgSO_4$ getrocknet und einrotiert. Nach Einengen der Lösung und Zugabe von Petrolether kristallisiert der gewünschte Ester aus. Ausbeute 2,8 g.

Beispiel 6

2-tert.-Butyloxycarbonylimino-3-tert. butoxycarbonyl-4-thiazolin-4-yl-essigsäure trimethylsilylethylester

Wird analog Beispiel 1 aus 2-Aminothiazol-4-yl-essigsäure-trimethylsilylester hergestellt. Ausbeute 50 %

Beispiel 7

1-(2-tert.-Butoxycarbonylaminothiazol-4-yl)-2-tert.-butoxy-carbonyloxypropancarbonsäureethylester

11,2 g (0,03 Mol) 2-tert.-Butoxycarbonylimino-3-tert.-Butoxycarbonyl-4-thiazolin-4-yl-essigsäureethylester wurden in 80 ml wasserfreiem Tetrahydrofuran gelöst, unter Stickstoff bei — 50 bis — 60 °C 20 ml (0,032 Mol) eine 15 %igen Lösung von n-Butyllithium in n-Hexan und anschließend 1.91 ml (0,034 Mol) Acetaldehyd hinzugegeben. Man rührt 2 h bei — 50 bis — 60 °C, gibt 30 ml einer 10 %igen Citronensäurelösung in Wasser hinzu und läßt den Ansatz auf Raumtemperatur erwärmen. Zur Aufarbeitung wird das Tetrahydrofuran i. Vak. bei Raumtemperatur abdestilliert, der Rückstand mit Methylenchlorid extrahiert, der org. Extrakt über $Na_2SO_4$ getrocknet und das Lösungsmittel abdestilliert. Man erhält 10,8 g eines Öls, das nach NMR ein Diasteromerengemisch ist (DC : Cyclohexan/Ether 1 : 1).

Beispiel 8

1-(2-tert.-Butoxycarbonylaminothiazol-4-yl)-1(E,Z)-propencarbonsäureethylester

Der Ansatz wird wie in Beispiel 7 angegeben angesetzt. Nach Zugabe des Acetaldehyds läßt man jedoch auf Raumtemperatur erwärmen, rührt über Nacht und arbeitet erst dann wie in Beispiel 7 angegeben auf. Man erhält 9,2 g eines Öls, das nach NMR und DC (Cyclohexan/Ether 1 : 1, Z-Isomer läuft höher) ein ~ 1 : 1 E/Z-Isomerengemisch ist. Die beiden Verbindungen lassen sich an Kieselgel 60 (Laufmittel Cyclohexan/Ether 1 : 1) trennen.

Z-Isomer :

$^1$H-NMR (250 MHz, $CDCl_3$) : δ = 10.5 (bs ; 1H, NH), 6.95 (s ; 1H, S—CH), 6.88 (q ; J = 7 Hz, 1H, CH—$CH_3$), 4.35 (q ; J = 7 Hz, 2H, CH—$CH_3$), 2.04 (d, J = 7 Hz, 3H CH—$CH_3$), 1.50 (s ; 9H, $C(CH_3)_3$), 1.36 (t ; J = 7 Hz, 3H, $CH_2$—$CH_3$).

E-Isomer :

$^1$H-NMR (250 MHz, $CDCl_3$) : δ = 10,5 (bs ; 1H, NH), 7.22 (q ; J = 7 Hz, 1H, CH—$CH_3$), 6.94 (s ; 1H, S—CH), 4.19 (q ; J = 7 Hz, 2H, $CH_2$—$CH_3$), 1,95 (d ; J = 7 Hz, 3H, CH—$CH_3$), 1.52 (s ; 9H, $C(CH_3)_3$), 1.22 (t ; J = 7 Hz, 3H, $CH_2$—$CH_3$).

Beispiel 9

2-(2-tert-Butoxycarbonylaminothiazol-4-yl)-2(E,Z)-benzylidenessigsäureethylester

3,86 g (0,01 Mol) 2-tert.-Butoxycarbonylimino-3-tert.-butoxycarbonyl-4-thiazolin-4-yl-essigsäureethylester in 40 ml wasserfreiem Tetrahydrofuran werden auf — 50° abgekühlt, 2,8 g (0,024 Mol) Kalium-tert.-butylat hinzugegeben, bis zur vollständigen Lösung gerührt und 1,11 ml (0,012 Mol) Benzaldehyd hinzugefügt. Man läßt den Ansatz auf Raumtemperatur erwärmen und rührt über Nacht.

Zur Aufarbeitung gibt man unter Eiskühlung und pH-Kontrolle etwa 12 ml 2 N HCl hinzu bis ein pH von 4-5 erreicht ist, zieht das Tetrahydrofuran und anschließend das tert.-Butanol i. Vak. ab und extrahiert den Rückstand mit Methylenchlorid. Nach dem Trocknen über $Na_2SO_4$ wird das Methylenchlorid abgezogen. Man erhält 3,1 g eines Öls, das nach NMR und DC (Cyclohexan/Ether 1 : 1) ein ~ 1 : 1 E/Z-Isomerengemisch ist.

Beispiel 10

1-(2-tert.-Butoxycarbonylaminothiazol-4-yl)-1-(Z)-propencarbonsäure

0,145 Mol (56 g) 2-tert.-Butoxycarbonylimino-3-tert.-butoxycarbonyl-4-thiazolin-4-yl-essigsäureethylester und 400 ml wasserfreies Tetrahydrofuran werden unter Stickstoff vorgelegt und bei — 60 bis — 50 °C 0,16 Mol n-Butyllithium in Hexan (15 %ig, 100 ml) zugetropft. Anschließend gibt man sofort 9,55 ml (0,17 Mol) Acetaldehyd hinzu, rührt 10 min. bei — 60 °C und anschließend über Nacht bei Raumtemperatur.

Dann gibt man 250 ml 2 N Natronlauge hinzu und rührt das Zweiphasengemisch intensiv 24 h bei Raumtemperatur. Anschließend wird das Tetrahydrofuran i. Vak. bei Raumtemperatur abdestilliert, die alkalische Phase 2 × mit je 100 ml Methylenchlorid extrahiert. Aus der wäßrigen Phase erhält man durch Ansäuern auf pH 2-3 und Extraktion die 1-(2-tert.-Butoxycarbonylaminothiazol-4-yl)-1(E)-propencarbonsäure (21,0 g, 51 %, Fp. = 195 °C (aus Acetonitril)).

Die Methylenchlorid-Phase wird i. Vak. eingedampft, der Rückstand in 250 ml Ethanol aufgenommen, mit 250 ml 2 N Natronlauge versetzt und 24 h auf 60 °C erhitzt.

Nach dem Abdestillieren des Ethanols wird die alkalische Phase 1 × mit 100 ml Methylenchlorid extrahiert, der Extrakt verworfen, die alkalische Phase auf pH 2-3 angesäuert und die gewünschte 1-(2-tert.-Butoxycarbonylaminothiazol-4-yl-1(Z)-propencarbonsäure mit Methylenchlorid extrahiert. (8,3 g, 20 %, Fp. = 183 °C (aus Acetonitril)).

Beispiel 11

1-(2-tert.-Butoxycarbonylaminothiazol-4-yl)-1(Z)-butencarbonsäure

Herstellung analog Beispiel 10 mit Propanal statt Acetaldehyd (Ausbeute 17 %, Fp. 172 °C, aus Acetonitril).

Beispiel 12

1-(2-tert.-Butoxycarbonylaminothiazol-4-yl)-1(Z)-pentencarbonsäure

Herstellung analog Beispiel 10 mit Butanal statt Acetaldehyd (Fp. 162-3 °C, aus Acetonitril).

Beispiel 13

1-(2-tert.-Butoxycarbonylaminothiazol-4-yl)-1(Z)-hexencarbonsäure

Herstellung analog Beispiel 10 mit Pentanal statt Acetaldehyd (Fp. 158 °C, aus Acetonitril).

Beispiel 14

1-(2-tert.-Butoxycarbonylaminothiazol-4-yl)-1(Z)-heptencarbonsäure

Herstellung analog Beispiel 10 mit Hexanal statt Acetaldehyd (Fp. 130-1 °C, aus Acetonitril).

Beispiel 15

1-(2-tert.-Butoxycarbonylaminothiazol-4-yl)-1(Z)-octencarbonsäure

Herstellung analog Beispiel 10 mit Heptanal statt Acetaldehyd (Fp. 164 °C, aus Acetonitril).

Beispiel 16

1-(2-tert.-Butoxycarbonylaminothiazol-4-yl)-3-methyl-1-(Z)-butencarbonsäure

Herstellung analog Beispiel 10 mit iso-Butyraldehyd statt Acetaldehyd (Fp. 169-71 °C, aus Acetonitril).

Beispiel 17

1-(2-tert.-Butoxycarbonylaminothiazol-4-yl)-4-methyl-1(Z)-pentencarbonsäure

Herstellung analog Beispiel 10 mit iso-Valeraldehyd statt Acetaldehyd (Fp. 173 °C, aus Acetonitril).

Beispiel 18

2-(2-tert.-Butoxycarbonylaminothiazol-4-yl)-3-cyclohexyl-(Z)-acrylsäure

Herstellung analog Beispiel 10 mit Cyclohexylaldehyd statt Acetaldehyd (Fp. > 210 °C, als Acetonitril).

Beispiel 19

1-(2-tert.-Butoxycarbonylaminothiazol-4-yl)-4-phenyl-1(Z)-butencarbonsäure

Herstellung analog Beispiel 10 mit Dihydrozimtaldehyd statt Acetaldehyd (Fp. 174 °C, aus Acetonitril).

Beispiel 20

1-(2-tert.-Butoxycarbonylaminothiazol-4-yl)-1(Z)-propencarbonsäure

0,43 Mol (122 g) 1-(2-tert.-Butoxycarbonylaminothiazol-4-yl)-1(E)-propencarbonsäure werden in 800 ml wasserfreiem Tetrahydrofuran mit 0,52 Mol (129 ml) Bistrimethylsilylacetamid versetzt und 1 h bei Raumtemperatur gerührt. Dann kühlt man auf —60 °C ab, gibt 1.74 Mol (200 g) Kalium-tert.-butylat (98 %ig) hinzu, läßt auf Raumtemperatur erwärmen und rührt über Nacht bei Raumtemperatur.

Zur Aufarbeitung gibt man unter Eiskühlung 100 ml Wasser hinzu, stellt mit etwa 900 ml 2 N HCl einen pH von 6-8 ein, zieht das Tetrahydrofuran i. Vak. ab, stellt einen pH von 2-3 ein und extrahiert 3 × mit 300 ml Methylenchlorid. Der Extrakt wird getrocknet, einrotiert und der Rückstand in 700 ml Methanol gelöst. Die methanolische Lösung gibt man mit einer Geschwindigkeit von etwa 10 ml/min über eine Säule (2,5 × 80 cm ; 400 ml) mit schwach basischem Ionenaustauscher Lewatit MP 62, wäscht mit 2 l Methanol und eluiert mit 1 l Methanol/2 N Natronlauge 10 :1. Das Eluat wird eingeengt, auf pH 2-3 mit 2 N HCl angesäuert und mit Methylenchlorid extrahiert. Nach dem Trocknen über $Na_2SO_4$ und Abdestillieren des Methylenchlorids erhält man 50 g (41 %) der gewünschten Z-Propencarbonsäure. Durch Eindampfen des methanolischen Ablaufs von der Säule erhält man die nicht isomerisierte E-Propencarbonsäure zurück.

Beispiel 21

1-(2-tert.-Butoxycarbonylaminothiazol-4-yl)-1(Z)-pentencarbonsäure

Durch Isomerisierung der entsprechenden E-Pentencarbonsäuren analog Beispiel 20.
Ausbeute 45 %.

Beispiel 22

1-(2-tert-Butoxycarbonylaminothiazol-4-yl)-1(Z)-propencarbonsäuremethansulfonsäureanhydrid

0,005 Mol (1,42 g) 1-(2-tert.-Butoxycarbonylaminothiazol-4-yl)-1(Z)-propencarbonsäure und 0,005 5 Mol (0,76 ml) Triethylamin werden in 10 ml wasserfreiem Tetrahydrofuran gelöst und auf —50 °C abgekühlt. Dann gibt man 0,005 1 Mol (0,40 ml) Methansulfonsäurechlorid hinzu und rührt 5 h bei —40 bis —50 °C. Anschließend wird unter $H_2O$-Ausschluß das Triethylaminhydrochlorid abgesaugt und das Tetrahydrofuran i. Vak. bei —10 °C abdestilliert. Man erhält das gemischte Anhydrid als Öl, das beim Erwärmen leicht in die E-Form isomerisiert (NMR).

Beispiel 23

1-(2-tert.-Butoxycarbonylaminothiazol-4-yl)-1(7)-butencarbonsäure-p-toluolsulfonsäureanhydrid

Herstellung analog Beispiel 22 aus entsprechenden Z-Butencarbonsäure und p-Toluolsulfochlorid bei —20 bis —30 °C.

Beispiel 24

7-[1-(2-tert.-Butoxycarbonylaminothiazol-4-yl)-1(Z)-propencarboxamido]-3-acetoxymethyl-3-cephem-4-carbonsäure

0,005 Mol (1,42 g) 1-(2-tert.-Butoxycarbonylaminothiazol-4-yl)-1(Z)-propencarbonsäure und 0,005 5 Mol (0,76 ml) Triethylamin werden in 20 ml wasserfreiem Methylenchlorid gelöst, auf —50 °C abgekühlt, 0,005 1 Mol (0,40 ml) Methansulfonsäurechlorid hinzugegeben und 5 h bei —50 bis —40 °C gerührt.

Dann gibt man eine auf —50 °C vorgekühlte Lösung von 0,006 Mol (1,63 g) 3-Acetoxymethyl-7-amino-3-cephem-4-carbonsäure und 0,013 Mol (1.80 ml) Triethylamin in 20 ml wasserfreiem Methylenchlorid hinzu, läßt innerhalb von 12 h auf Raumtemperatur erwärmen.

Zur Aufarbeitung wird der Ansatz zweimal mit je 10 ml $H_2O$ gewaschen, die Methylenchlorid-Phase mit 40 ml $H_2O$ überschichtet und unter Rühren und Eiskühlung mit 1 N HCl auf pH 2-3 angesäuert. Die organische Phase wird abgetrennt, die $H_2O$-Phase 2 x mit je 20 ml Methylenchlorid extrahiert, die vereinigten Methylenchlorid-Phasen mit ges. NaCl-Lösung gewaschen, über $Na_2SO_4$ getrocknet und i. Vak. einrotiert. Man erhält das gewüschte Cephlosporin nahezu quantitativ.

Beispiel 25

7-[1-(2-tert.-Butoxycarbonylaminothiazol-4-yl)-1 (Z)-propencarboxamido]-3-(1-methyl-1-H-tetrazol-5-yl)thiomethyl-3-cephem-4-carbonsäure

Die Herstellung erfolgt analog Beispiel 24 aus 1-(2-tert.-Butoxycarbonylaminothiazol-4-yl)-1(Z)-propencarbonsäure und 7-Amino-3-(1-methyl-1-H-tetrazol-5-yl)thiomethyl-3-cephem-4-carbonsäure. Ausbeute 92 %.

Beispiel 26

7-[1-(2-tert.-Butoxycarbonylaminothiazol-4-yl)-1(Z)-butencarboxyamido]-3-acetoxymethyl-3-cephem-4-carbonsäure

Herstellung analog Beispiel 24 aus 1-(2-tert.-Butoxycarbonylaminothiazol-4-yl)-1-(Z)-butencarbonsäure und 3-Acetoxymethyl-7-amino-3-cephem-4-carbonsäure.

Beispiel 27

7-[1-(2-tert.-Butoxycarbonylaminothiazol-4-yl)-1(Z)-butencarboxamido]-3-(1-methyl-1-H-tetrazol-5-yl)thiomethyl-3-cephem-4-carbonsäure

Herstellung analog Beispiel 24 aus 1-tert.-Butoxycarbonylaminothiazol-4-yl)-1(Z)-butencarbonsäure und 7-Amino-3-(1-methyl-1-H-tetrazol-5-yl)thiomethyl-3-cephem-4-carbonsäure. Ausbeute 88 %.

Beispiel 28

7-[1-(2-tert.-Butoxycarbonylaminothiazol-4-yl)-1(Z)-hepten-carboxamido]-3-acetoxymethyl-3-cephem-4-carbonsäure

Herstellung analog Beispiel 24 aus 1-(2-tert.-Butoxycarbonylaminothiazol-4-yl)-1(Z)-heptencarbonsäure und 3-Acetoxymethyl-7-amino-3-cephem-4-carbonsäure. Ausbeute 90 %.

Beispiel 29

7-[1-(2-tert.-Butoxycarbonylaminothiazol-4-yl)-1(Z)-hepten-carboxamido]-3-(1-methyl-1-H-tetrazol-5-yl)-thiomethyl-3-cephem-4-carbonsäure

Herstellung analog Beispiel 24 aus 1-(2-tert.-Butoxycarbonylaminothiazol-4-yl)-1(Z)-heptencarbonsäure und 7-Amino-3-(1-methyltetrazol-5-yl)thiomethyl-3-cephem-4-carbonsäure.

Ausbeute 85 %.

Beispiel 30

7-[1-(2-tert.-Butoxycarbonylaminothiazol-4-yl)-3-methyl-1(Z)-butencarboxamido]-3-acetoxymethyl-3-cephem-4-carbonsäure

Herstellung analog Beispiel 24 aus 1-(2-tert.-Butoxycarbonylaminothiazol-4-yl)-3-methyl-1(Z)-butencarbonsäure und 3-Acetoxymethyl-7-amino-3-cephem-4-carbonsäure.

Ausbeute 93 %.

Beispiel 31

7-[1 (2-tert.-Butoxycarbonylaminothiazol-4-yl)-4-phenyl-1(Z)-butencarboxamido]-3-acetoxymethyl-3-cephem-4-carbonsäure

Herstellung analog Beispiel 24 aus 1-(2-tert.-Butoxycarbonylaminothiazol-4-)-4-phenyl-1(Z)-butensäure und 3-Acetoxymethyl-7-amino-3-cephem-4-carbonsäure. Ausbeute 95 %.

Beispiel 32

7-[1-(2-tert.-Butoxycarbonylaminothiazol-4-yl)-1(Z)-propancarboxamido]-3-methyl-3-cephem-4-carbonsäure

Herstellung analog Beispiel 24 aus 1-(2-tert.-Butoxycarbonylaminothiazol-4-yl)-1(Z)-propencarbonsäure und 7-Amino-3-methyl-3-cephem-4-carbonsäure. Im Unterschied zu Beispiel 24 wird die 7-Amino-3-methyl-3-cephem-4-carbonsäure statt mit Triethylamin mit der äquimolaren Menge Diisopropylamin in Methylenchlorid gelöst.

Ausbeute 88 %.

Beispiel 33

7-[1-(2-tert.-Butoxycarbonylaminothiazol-4-yl)-1(Z)-propencarboxamido]-3-aminocarbonyloxymethyl-3-cephem-4-carbonsäure

Herstellung analog Beispiel 24 aus 1-(2-tert.-Butoxycarbonyl-aminothiazol-4-yl)-1(Z)-propencarbonsäure und 7-Amino-3-aminocarbonyloxymethyl-3-cephem-4-carbonsäure. Im Unterschied zu Beispiel 24 wird die 7-Amino-3-aminocarbonyloxymethyl-3-cephem-4-carbonsäure nicht mit Triethylamin in Methylenchlorid, sondern mit der äquimolaren Menge Diisopropylamin in wasserfreiem Dimethylformamid gelöst und die erhaltene Lösung zu dem gemischten Carbon-Sulfonsäureanhydrid in Methylenchlorid gegeben.

Zur Aufarbeitung wird der Ansatz i. Vak. bei 0 °C eingedampft, der Rückstand in Wasser aufgenommen, mit Methylenchlorid extrahiert, die wäßrige Phase mit Essigester überschichtet und auf pH 2-3 angesäuert. Das Produkt scheidet sich als Öl zwischen den Phasen ab.

Beispiel 34

7-[1-(2-tert.-Butoxycarbonylaminothiazol-4-yl)-1(Z)-propencarboxamido]-3-cephem-4-carbonsäurediphenyl-methylester

Herstellung analog Beispiel 24 aus 1-(2-tert.-Butoxy-carbonyl-aminothiazol-4-yl)-1(Z)-propencarbonsäure und 7-Amino-3-cephem-4-carbonsäurediphenylmethylester. Ausbeute 93 %.

Beispiel 35

7-[1-(2-Aminothiazol-4-yl)-1(Z)-propencarboxamido]-3-acetoxymethyl-3-cephem-4-carbonsäure

Zu dem BOC-geschützten Cephalosporin aus Beispiel 24 gibt man 10 ml Trifluoressigsäure und rührt 30 min bei Raumtemperatur. Dann wird die Trifluoressigsäure i. Vak. bei Raumtemperatur abgezogen, zum Rückstand 20 ml Methanol/$H_2O$ 10 : 1 und anschließend 10 %ige Na $HCO_3$-Lösung hinzugegeben bis bei pH 6-7 eine klare Lösung entstanden ist. Dann stellt man langsam mit 1 N HCl einen pH von 33 ein, zieht das Methanol i. Vak. langsam ab und stellt gegebenenfalls den pH auf 3 nach. Das ausgefallene Produkt wird abgesaugt. Ausbeute 70 %.

Beispiele 36-44

Die Cephalosporine aus den Beispielen 25 bis 34 werden analog Beispiel 35 deblockiert. Die Ausbeuten liegen zwischen 50 und 90 %.

**Patentansprüche**

1. Verbindungen der Formel X

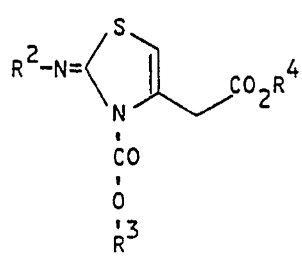

(X)

in der

$R^2$ $CO_2R^3$

$R^3$ und $R^4$ gleich oder verschieden sein können und ein gegebenenfalls substituierter Alkylrest mit 1-15 C-Atomen, ein gegebenenfalls substituierter Alkenylrest mit 3-15 C-Atomen, ein gegebenenfalls substituierter Cycloalkylrest mit 3-10 C-Atomen, ein gegebenenfalls substituierter Cycloalkenylrest mit 5-10 C-Atomen, ein gegebenenfalls substituierter Arylrest mit 1 bis 3 Ringen oder ein gegebenenfalls substituierter Heterocyclylrest mit 1-3 Ringen, die bis zu 5 Stickstoff-, Schwefel- oder Sauerstoffatome enthalten können, sind und die genannten Alkyl-, Alkenyl-, Cycloalkyl- und Cycloalkenylreste durch Alkylreste mit 1-4 C-Atomen, O-Alkylreste mit 1-4 C-Atomen, Halogen, gegebenenfalls substituierte Phenylreste, C ≡ M und $C_1$-$C_5$-Trialkylsilyl substituiert sein können und die Aryl- und Heterocyclylreste einschließlich der genannten Phenylreste durch Alkyl-, O-Alkyl-, S-Alkyl-, Alkyloxycarbonyl-, Halogen- und Phenylreste, sowie durch Nitro und C ≡ N substituiert sein können, wobei alle Alkylreste 1 bis 4 C-Atome haben.

2. Verbindungen nach Anspruch 1, dadurch gekennzeichnet, daß

$R^2$ O—CO—$C(CH_3)_3$

$R^3$ $C(CH_3)_3$

und

$R^4$ die in Anspruch 1 gegebene Bedeutung hat.

3. Verbindungen nach den Ansprüchen 1 und 2, dadurch gekennzeichnet, daß

$R^2$ tert.-Butoxycarbonyl

$R^3$ tert. Butyl und

$R^4$ Methyl oder Ethyl ist.

4. Verfahren zur Herstellung der Verbindungen der Formel X

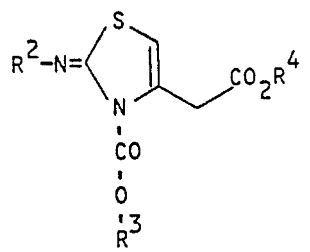

(X)

in denen

$R^2$ $CO_2R^3$

$R^3$ und $R^4$ gleich oder verschieden sein können und ein gegebenenfalls substituierter Alkylrest mit 1-15 C-Atomen, ein gegebenenfalls substituierter Alkenylrest mit 3-15 C-Atomen, ein gegebenenfalls substituierter Cycloalkylrest mit 3-10 C-Atomen, ein gegebenenfalls substituierter Cycloalkenylrest mit 5-10 C-Atomen, ein gegebenenfalls substituierter Arylrest mit 1 bis 3 Ringen oder ein gegebenenfalls substituierter Heterocyclylrest mit 1-3 Ringen, die bis zu 5 Stickstoff-, Schwefel- oder Sauerstoffatome enthalten können, sind und die genannten Alkyl-, Alkenyl-, Cycloalkyl- und Cycloalkenylreste durch Alkylreste mit 1-4 C-Atomen, O-Alkylreste mit 1-4 C-Atomen, Halogen, gegebenenfalls substituierte Phenylreste, $C \equiv N$ und $C_1$-$C_5$-Trialkylsilyl substituiert sein können und die Aryl- und Heterocyclylreste einschließlich der genannten Phenylreste durch Alkyl-, O-Alkyl-, S-Alkyl-, Alkyloxycarbonyl-, Halogen- und Phenylreste, sowie durch Nitro und $C \equiv N$ substituiert sein können, wobei alle Alkylreste 1 bis 4 C-Atome haben, dadurch gekennzeichnet, daß man eine Verbindung der Formel IX

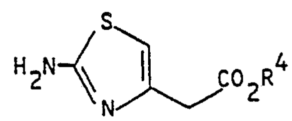

(IX)

mit einem Pyrokohlensäureester der Formel

$$R^3—O—CO—O—CO—O—R^3$$

in einem geeigneten Lösungsmittel umsetzt, wobei $R^3$ und $R^4$ die obengenannte Bedeutung haben.

5. Verbindungen der Formel XI

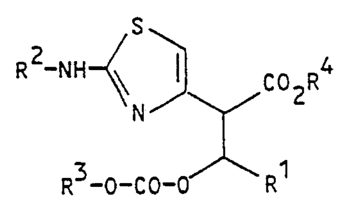

(XI)

in denen $R^2$, $R^3$ und $R^4$ die in den Ansprüchen 1-3 angegebene Bedeutung haben und

$R^1$ einen gegebenenfalls substituierten Alkylrest mit 1-15 C-Atomen, einen gegebenenfalls substituierten Cycloalkylrest mit 3-10 C-Atomen, einen gegebenenfalls substituierten Arylrest mit 1 bis 2 Ringen oder einen gegebenenfalls substituierten Heterocyclylrest mit 1 bis 3 Ringen, die bis zu 5 Stickstoff-, Schwefel- und Sauerstoffatome enthalten können, der durch darstellt, und die genannten Alkyl- und Cycloalkylreste durch Alkylreste mit 1-6 C-Atomen, O-Alkylreste mit 1-6 C-Atomen, S-Alkylreste mit 1-6 C-Atomen, N-Alkylreste mit 1-6 C-Atomen, Alkyloxycarbonylreste mit 1-6 C-Atomen und gegebenenfalls substituierte Phenylreste substituiert sein können und die Aryl- und Heterocyclylreste ein schließlich der genannten Phenylreste durch Alkyl-, O-Alkyl-, S-Alkyl-, Alkyloxycarbonyl-, Halogen-, vorzugsweise Chlor, und Phenylreste, wobei alle Alkylreste 1-6 C-Atome tragen, substituiert sein können gekennzeichnete Teil muß in Anspruch 5 und in Anspruch 6 aufscheinen.

6. Verfahren zur Herstellung von Verbindungen der Formel XI

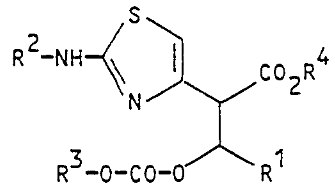

(XI)

in der

$R^2$, $R^3$ und $R^4$ die in Anspruch 4 gegebenen Bedeutungen haben, und

$R^1$ einen gegebenenfalls substituierten Alkylrest mit 1-15 C-Atomen, einen gegebenenfalls substituierten Cycloalkylrest mit 3-10 C-Atomen, einen gegebenenfalls substituierten Arylrest mit 1 bis 2 Ringen oder einen gegebenenfalls substituierten Heterocyclylrest mit 1 bis 3 Ringen, die bis zu 5 Stickstoff-, Schwefel- und Sauerstoffatome enthalten können, wie in Anspruch 5, dadurch gekennzeichnet, daß man Verbindungen der Formel X gemäß Anspruch 4 in einem geeigneten Lösungsmittel bei tiefen Temperaturen mit einer Base und anschließend mit einem Aldehyd der Formel

$$R^1—CHO$$

umsetzt, wobei $R^1$ wie oben definiert ist.

7. Verfahren nach Anspruch 6, dadurch gekennzeichnet, daß 1-1,1 Äquivalente des Aldehyds eingesetzt werden.

8. Verbindungen der Formel XVI

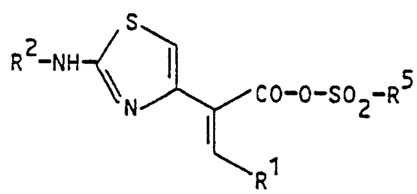

(XVI)

in der

$R^1$ und $R^2$ die in Anspruch 5 angegebene Bedeutung haben und

$R^5$ Fluor, einen gegebenenfalls substituierten Alkyl-, Alkenyl-, Cycloalkyl-, Cycloalkenyl-, carbocyclischen oder heterocyclischen Aryl- oder Heterocyclylrest darstellt.

9. Verbindungen gemäß Anspruch 8, dadurch gekennzeichnet, daß $R^5$ Methyl oder p-Tolyl bedeutet.

10. Verfahren zur Herstellung von Verbindungen der Formel XVI

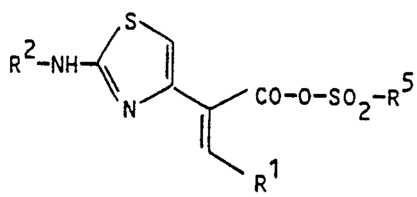

(XVI)

dadurch gekennzeichnet, daß man Verbindungen der Formel XIII

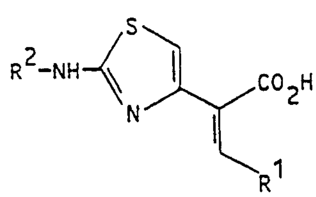

(XIII)

mit einer Verbindung der Formel XV

$$Z—SO_2—R^5$$

umsetzt, wobei

$R^1$ die in Anspruch 6 und

$R^2$ die in Anspruch 4 gegebenen Bedeutungen haben, und

$R^5$ Fluor, einen gegebenenfalls substituierten Alkyl-, Alkenyl-, Cycloalkyl-, Cycloalkenyl-, carbocyclischen oder heterocyclischen Aryl- oder Heterocyclylrest darstellt, und

Z für Cl, Br oder —O—$SO_2R^5$ steht.

11. Verfahren nach Anspruch 10, dadurch gekennzeichnet, daß man die Umsetzung in einem geeigneten Lösungsmittel in Anwesenheit eines geeigneten Amins bei Temperaturen zwischen Raumtemperatur und — 80 °C durchführt.

12. Verfahren zur Herstellung von Verbindungen der allgemeinen Formel I

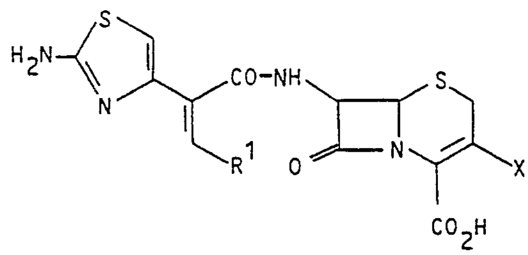

(I)

in der

$R^1$ die in Anspruch 6 gegebene Bedeutung hat und

X einen bei Cephalosporinen üblichen Rest darstellt, dadurch gekennzeichnet, daß man

a) Verbindungen der Formel IX

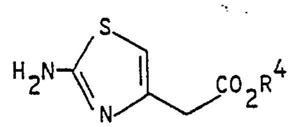

(IX)

mit einem Pyrokohlensäureester der Formel

$$R^3—O—CO—O—CO—O—R^3$$

umsetzt, wobei $R^3$ und $R^4$ wie in Anspruch 4 definiert sind,

b) das so erhaltene Produkt X

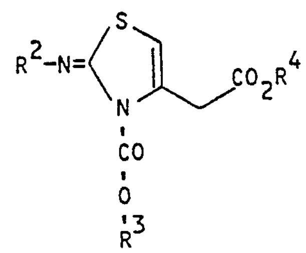

(X)

worin $R^2$ $CO_2R^3$ bedeutet und $R^3$ die obengenannte Bedeutung hat, zunächst mit einer geeigneten Base und dann mit einem Aldehyd der Formel $R^1$—CHO zu der Verbindung XI

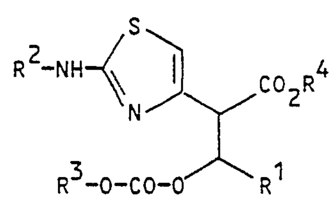

(XI)

umsetzt, wobei $R^1$ die oben angegebene Bedeutung hat ;

c) die Verbindung XI mit einer Base zur Verbindung XII umsetzt

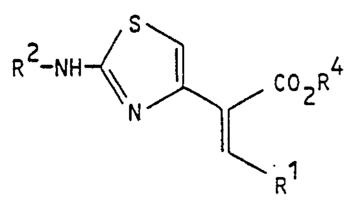

(XII)

d) aus XII durch Trennung von Z und E-Isomeren und anschließende Verseifung oder durch selektive Verseifung die Z-Säure XIII

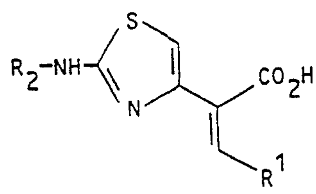

(XIII)

gewinnt ;

e) XIII mit einer Verbindung $Z—SO_2R^5$ zur Verbindung XVI

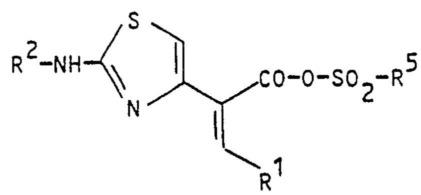

(XVI)

umsetzt, wobei $R^5$ und Z die in Anspruch 10 gegebenen Bedeutungen haben ;

f) XVI mit einer Cephalosporansäure der Formel XVII

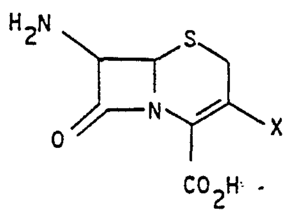

worin X die obengenannte Bedeutung hat, kuppelt, und

g) die Schutzgruppe $R^2$ abspaltet.

**Claims**

1. Compounds of the formula X

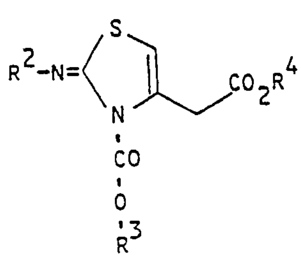

(X)

in which

$R^2$ $CO_2R^3$ and

$R^3$ and $R^4$ can be identical or different and are an optionally substituted alkyl radical with 1-15 C atoms, an optionally substituted alkenyl radical with 3-15 C atoms, an optionally substituted cycloalkyl radical with 3-10 C atoms, an optionally substituted cycloalkenyl radical with 5-10 C atoms, an optionally substituted aryl radical with 1 to 3 rings or an optionally substituted heterocyclyl radical with 1-3 rings which can contain up to 5 nitrogen, sulphur or oxygen atoms and the abovementioned alkyl, alkenyl, cycloalkyl and cycloalkenyl radicals can be substituted by alkyl radicals with 1-4 C atoms, O-alkyl radicals with 1-4 C atoms, halogen, optionally substituted phenyl radicals, C ≡ N and $C_1$-$C_5$-trialkylsilyl and the aryl and heterocyclyl radicals, including the abovementioned phenyl radicals, can be substituted by alkyl, O-alkyl, S-alkyl, alkyloxycarbonyl, halogen and phenyl radicals, and by nitro and C ≡ N, all the alkyl radicals having 1 to 4 C atoms.

2. Compounds according to Claim 1, characterised in that

$R^2$ O—CO—C$(CH_3)_3$
$R^3$ C$(CH_3)_3$
and
$R^4$ has the meaning given in Claim 1.

3. Compounds according to Claims 1 and 2, characterised in that
$R^2$ is tert.-butoxycarbonyl,
$R^3$ is tert.-butyl
and
$R^4$ is methyl or ethyl.

4. Process for the preparation of the compounds of the formula X

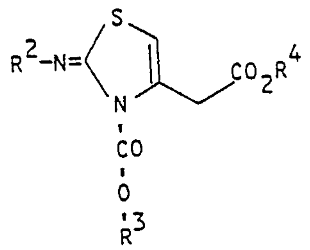

(X)

in which
$R^2$ $CO_2R^3$ and
$R^3$ and $R^4$ can be identical or different and are an optionally substituted alkyl radical with 1-15 C atoms, an optionally substituted alkenyl radical with 3-15 C atoms, an optionally substituted cycloalkyl radical with 3-10 C atoms, an optionally substituted cycloalkenyl radical with 5-10 C atoms, an optionally substituted aryl radical with 1 to 3 rings or an optionally substituted heterocyclyl radical with 1-3 rings which can contain up to 5 nitrogen, sulphur or oxygen atoms, and the abovementioned alkyl, alkenyl, cycloalkyl and cycloalkenyl radicals can be substituted by alkyl radicals with 1-4 C atoms, O-alkyl radicals with 1-4 C atoms, halogen, optionally substituted phenyl radicals, C ≡ N and $C_1$-$C_5$-trialkylsilyl and the aryl and heterocyclyl radicals, including the abovementioned phenyl radicals, can be substituted by alkyl, O-alkyl, S-alkyl, alkyloxycarbonyl, halogen and phenyl radicals, and by nitro and C ≡ N, all the alkyl radicals having 1 to 4 C atoms, characterised in that a compound of the formula IX

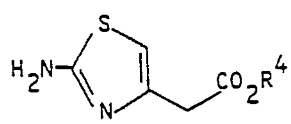

(IX)

is reacted with a pyrocarbonic acid ester of the formula

$R^3$—O—CO—O—CO—O—$R^3$

in a suitable solvent, $R^3$ and $R^4$ having the abovementioned meaning.

5. Compounds of the formula XI

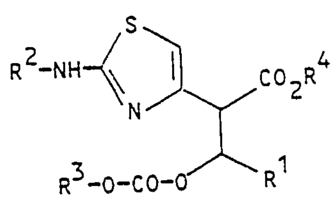

(XI)

in which
$R^2$, $R^3$ and $R^4$ have the meaning given in Claims 1-3 and
$R^1$ represents an optionally substituted alkyl radical with 1-15 C atoms, an optionally substituted cycloalkyl radical with 3-10 C atoms, an optionally substituted aryl radical with 1 to 2 rings or an optionally substituted heterocyclyl radical with 1 to 3 rings which can contain up to 5 nitrogen, sulphur and oxygen atoms, and the abovementioned alkyl and cycloalkyl radicals can be substituted by alkyl radicals with 1-6 C atoms, O-alkyl radicals with 1-6 C atoms, S-alkyl radicals wit 1-6 C atoms, N-alkyl radicals with 1-6 C atoms, alkyloxycarbonyl radicals with 1-6 C atoms and optionally substituted phenyl radicals and the aryl and heterocyclyl radicals, including the abovementioned phenyl radicals, can be substituted by alkyl, O-alkyl, S-alkyl, alkyloxycarbonyl, halogen, preferably chlorine, and phenyl radicals, all the alkyl radicals carrying 1-6 C atoms.

6. Process for the preparation of compounds of the formula XI

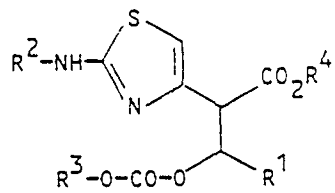

(XI)

in which

$R^2$, $R^3$ and $R^4$ have the meanings given in Claim 4, and

$R^1$ represents an optionally substituted alkyl radical with 1-15 C atoms, an optionally substituted cycloalkyl radical with 3-10 C atoms, an optionally substituted aryl radical with 1 to 2 rings or an optionally substituted heterocyclyl radical with 1 to 3 rings which can contain up to 5 nitrogen, sulphur and oxygen atoms, and the abovementioned alkyl and cycloalkyl radicals can be substituted by alkyl radicals with 1-6 C atoms, O-alkyl radicals with 1-6 C atoms, S-alkyl radicals with 1-6 C atoms, N-alkyl radicals with 1-6 C atoms, alkyloxycarbonyl radicals with 1-6 C atoms and optionally substituted phenyl radicals and the aryl and heterocyclyl radicals, including the abovementioned phenyl radicals, can be substituted by alkyl, O-alkyl, S-alkyl, alkyloxycarbonyl, halogen, preferably chlorine, and phenyl radicals, all the alkyl radicals carrying 1-6 C atoms, characterised in that compounds of the formula X according to Claim 4 are reacted, in a suitable solvent at low temperatures, with a base and then with an aldehyde of the formula

$$R^1—CHO$$

wherein $R^1$ is as defined above.

7. Process according to Claim 6, characterised in that 1-1.1 equivalents of the aldehyde are used.

8. Compounds of the formula XVI

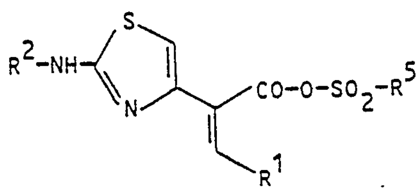

(XVI)

in which

$R^1$ and $R^2$ have the meaning given in Claim 5 and

$R^5$ represents fluorine, an optionally substituted alkyl, alkenyl, cycloalkyl, cycloalkenyl, carbocyclic or heterocyclic aryl or heterocyclyl radical.

9. Compounds according to Claim 8, characterised in that $R^5$ denotes methyl or p-tolyl.

10. Process for the preparation of compounds of the formula XVI

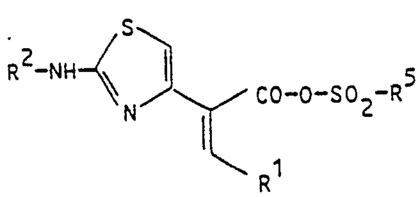

(XVI)

characterised in that compounds of the formula XIII

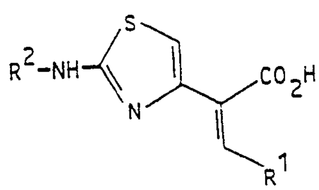

(XIII)

are reacted with a compound of the formula XV

$$Z—SO_2—R^5$$

wherein

$R^1$ has the meaning given in Claim 6 and

$R^2$ has the meaning given in Claim 4 and

$R^5$ represents fluorine, an optionally substituted alkyl, alkenyl, cycloalkyl, cycloalkenyl, carbocyclic or heterocyclic aryl or heterocyclyl radical and

Z represents Cl, Br or $-O-SO_2R^5$.

11. Process according to Claim 10, characterised in that the reaction is carried out in a suitable solvent in the presence of a suitable amine at temperatures between room temperature and −80 °C.

12. Process for the preparation of compounds of the general formula I

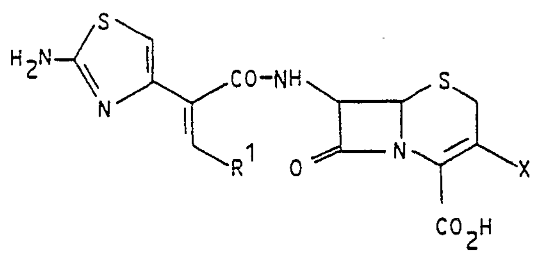

(I)

in which

$R^1$ has the meaning given in Claim 6 and

X represents a radical customary in cephalosporins, characterised in that

a) compounds of the formula IX

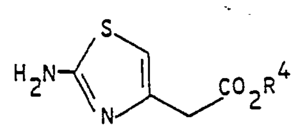

(IX)

are reacted with a pyrocarbonic acid ester of the formula

$$R^3-O-CO-O-CO-O-R^3$$

wherein $R^3$ and $R^4$ are as defined in Claim 4,

b) the resulting product X

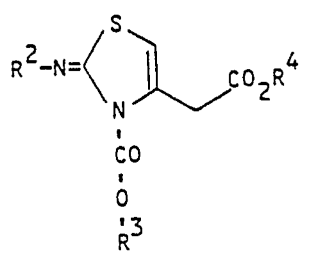

(X)

wherein $R^2$ denotes $CO_2R^3$ and $R^3$ has the abovementioned meaning, is reacted first with a suitable base and then with an aldehyde of the formula $R^1-CHO$ to give the compound XI

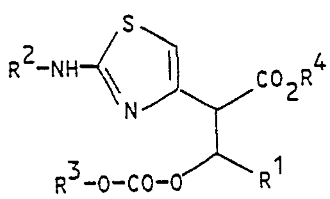

(XI)

wherein $R^1$ has the abovementioned meaning ;

c) the compound XI is reacted with a base to give the compound XII

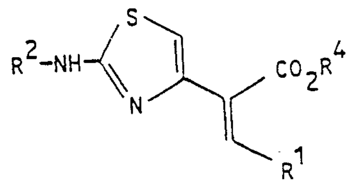

(XII)

d) the Z-acid XIII

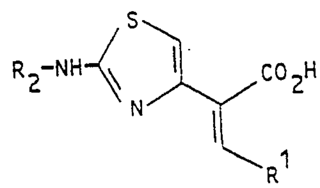

(XIII)

is obtained from XII by separation of the Z and E isomers and subsequent saponification or by selective saponification ;

e) XIII is reacted with a compound $Z—SO_2R^5$ to give the compound XVI

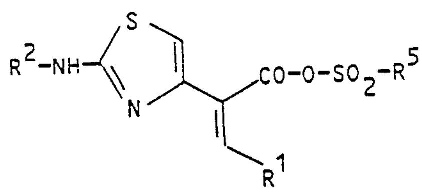

(XVI)

wherein $R^5$ and Z have the meanings given in Claim 10 ;

f) XVI is coupled with a cephalosporanic acid of the formula XVII

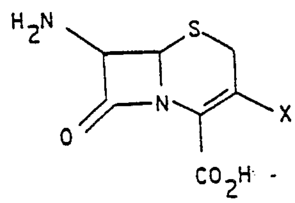

wherein X has the abovementioned meaning and

g) the protective group $R^2$ is split off.

**Revendications**

1. Composés de formule X

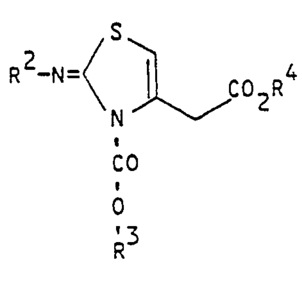

(X)

dans laquelle

$R^2$ représente $CO_2R^4$

$R^3$ et $R^4$ peuvent être identiques ou différents et représentent chacun un reste alkyle ayant 1 à 15 atomes, éventuellement substitué, un reste alcényle ayant 3 à 15 atomes de carbone, éventuellement substitué, un reste cycloalkyle ayant 3 à 10 atomes de carbone, éventuellement substitué, un reste cycloalcényle ayant 5 à 10 atomes de carbone, éventuellement substitué, un reste aryle ayant 1 à 3 noyaux

éventuellement substitué, ou un reste hétérocyclyle ayant 1 à 3 noyaux, éventuellement substitué, qui peuvent contenir jusqu'à 5 atomes d'azote, de soufre ou d'oxygène, et les restes alkyle, alcényle, cycloalkyle et cycloalcényle précités peuvent être substitués par des restes alkyles ayant 1 à 4 atomes de carbone, des restes O-alkyles ayant 1 à 4 atomes de carbone, de l'halogène, des restes phényles éventuellement substitués, C ≡ N et, un reste trialkyle (en $C_1$ à $C_5$) silyle, et les restes aryle et hétérocyclyle, y compris les restes phényles cités, peuvent être substitués par des restes-alkyle, O-alkyle, S-alkyle, alkyloxycarbonyle, halogéno et phényle, ainsi que par un groupe nitro et C ≡ N, tous les restes alkyles ayant 1 à 4 atomes de carbone.

2. Composés selon la revendication 1, caractérisé en ce que :

$R^2$ représente O—CO—$C(CH_3)_3$

$R^3$ représente $C(CH_3)_3$

et

$R^4$ a le sens indiqué à la revendication 1.

3. Composés selon les revendications 1 et 2, caractérisés en ce que

$R^2$ représente un groupe tertiobutoxycarbonyle,

$R^3$ représente un groupe tertiobutyle,

et

$R^4$ représente un groupe méthyle ou éthyle.

4. Procédé pour préparer les composés de formule X

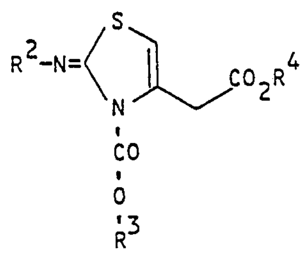

(X)

dans laquelle

$R^2$ représente $CO_2R^4$

$R^3$ et $R^4$ peuvent être identiques ou différents et représentent chacun un reste alkyle ayant 1 à 15 atomes, éventuellement substitué, un reste alcényle ayant 3 à 15 atomes de carbone, éventuellement substitué, un reste cycloalkyle ayant 3 à 10 atomes de carbone, éventuellement substitué, un reste cycloalcényle ayant 5 à 10 atomes de carbone, éventuellement substitué, un reste aryle ayant 1 à 3 noyaux éventuellement substitué, ou un reste hétérocyclyle ayant 1 à 3 noyaux, éventuellement substitué, qui peuvent contenir jusqu'à 5 atomes d'azote, de soufre ou d'oxygène, et les restes alkyle, alcényle, cycloalkyle et cycloalcényle précités peuvent être substitués par des restes alkyles ayant 1 à 4 atomes de carbone, des restes O-alkyles ayant 1 à 4 atomes de carbone, de l'halogène, des restes phényles éventuellement substitués, C ≡ N et un reste trialkyle (en $C_1$ à $C_5$) silyle, et les restes aryle et hétérocyclyle, y compris les restes phényles cités, peuvent être substitués par des restes alkyle, O-alkyle, S-alkyle, alkyloxycarbonyle, halogéno et phényle, ainsi que par un groupe nitro et C ≡ N, d'où les restes alkyles ayant 1 à 4 atomes de carbone, procédé caractérisé en ce qu'on fait réagir un composé de formule IX

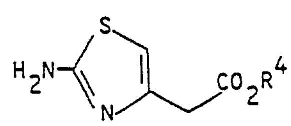

(IX)

avec un ester d'acide pyrocarbonique de formule

$$R^3—O—CO—O—CO—O—R^3$$

dans un solvant convenable, $R^3$ et $R^4$ ayant le sens précité.

5. Composés de formule XI

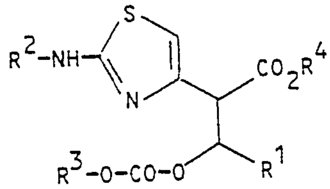

(XI)

dans lesquels

$R^2$, $R^3$ et $R^4$ ont le sens indiqué aux revendications 1 à 3, et

$R^1$ représente un reste alkyle ayant 1 à 15 atomes de carbone, éventuellement substitué, un reste cycloalkyle ayant 3 à 10 atomes de carbone, éventuellement substitué, un reste aryle ayant 1 ou 2 noyaux, éventuellement substitué, ou un reste hétérocyclyle comportant 1 à 3 noyaux, éventuellement substitué, qui peuvent contenir jusqu'à 5 atomes d'azote, de soufre et d'oxygène, et les restes alkyle et cycloalkyle peuvent être substitués par des restes alkyles ayant 1 à 6 atomes de carbone, des restes O-alkyles ayant 1 à 6 atomes de carbone, des restes S-alkyles ayant 1 à 6 atomes de carbone, des restes 1-alkyles ayant 1 à 6 atomes de carbone, des restes alkyloxycarbonyles ayant 1 à 6 atomes de carbone et des restes phényles éventuellement substitués, et les restes aryle et hétérocyclyle, y compris les restes phényles cités, peuvent être substitués par des restes alkyle, O-alkyle, S-alkyle, alkyloxycarbonyle, halogéno, de préférence chloro, et des restes phényles, tous les restes alkyles comportant 1 à 6 atomes de carbone.

6. Procédé pour préparer des composés de formule XI :

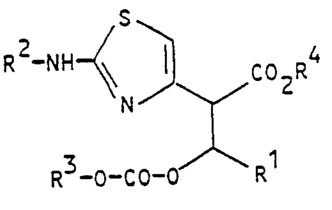

(XI)

dans laquelle

$R^2$, $R^3$ et $R^4$ ont le sens indiqué aux revendications 1 à 3, et

$R^1$ représente un reste alkyle ayant 1 à 15 atomes de carbone, éventuellement substitué, un reste cycloalkyle ayant 3 à 10 atomes de carbone, éventuellement substitué, un reste aryle ayant 1 ou 2 noyaux, éventuellement substitué, ou un reste hétérocyclyle comportant 1 à 3 noyaux, éventuellement substitué, qui peuvent contenir jusqu'à 5 atomes d'azote, de soufre et d'oxygène, et les restes alkyle et cyloalkyle peuvent être substitués par des restes alkyles ayant 1 à 6 atomes de carbone, des restes O-alkyles ayant 1 à 6 atomes de carbone, des restes S-alkyles ayant 1 à 6 atomes de carbone, des restes 1-alkyles ayant 1 à 6 atomes de carbone, des restes alkyloxycarbonyles ayant 1 à 6 atomes de carbone et des restes phényles éventuellement substitués, et les restes aryle et hétérocyclyle, y compris les restes phényles cités, peuvent être substitués par des restes alkyle, O-alkyle, S-alkyle, alkyloxycarbonyle, halogéno, de préférence chloro, et des restes phényles, tous les restes alkyles comportant 1 à 6 atomes de carbone, procédé caractérisé en ce qu'on fait réagir les composés de formule X selon la revendication IV, dans un solvant convenable à de basses températures, avec une base puis avec un aldéhyde de formule

$$R^1—CHO$$

$R^1$ étant tel que défini ci-dessus.

7. Procédé selon la revendication 6, caractérisé en ce qu'on utilise 1 à 1,1 équivalent de l'aldéhyde.

8. Composés de formule XVI

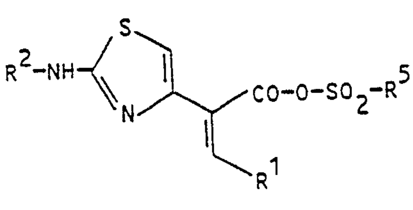

(XVI)

dans laquelle

$R^1$ et $R^2$ ont le sens indiqué à la revendication 5, et $R^5$ représente un atome de fluor, un reste alkyle, alcényle, cycloalkyle, cycloalcényle, aryle carbocyclique ou hétérocyclique hétérocyclyle, éventuellement substitués.

9. Composés selon la revendication 8, caractérisé en ce que $R^5$ représente un groupe méthyle ou p-tolyle.

10. Procédé pour préparer des composés de formule XVI

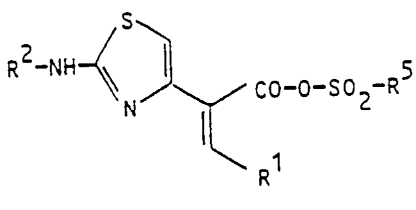

(XVI)

caractérisé en ce qu'on fait réagir des composés de formule XIII

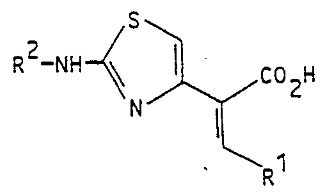

(XIII)

avec un composé de formule XV

$$Z—SO_2—R^5$$

formules dans lesquelles

$R^1$ a les sens indiqués à la revendication 6 et

$R^2$ a les sens indiqués à la revendication 4, et

$R^5$ représente un atome de fluor, un groupe alkyle, alcényle, cycloalkyle, cycloalcényle, aryle, carbocyclique ou hétérocyclique ou hétérocyclyle éventuellement substitués, et Z représente Cl, Br ou $—O—SO_2R^5$.

11. Procédé selon la revendication 10, caractérisé en ce qu'on conduit la réaction dans un solvant convenable en présence d'une amine convenable à des températures comprises entre la température ambiante et — 80 °C.

12. Procédé pour préparer des composés de formule générale

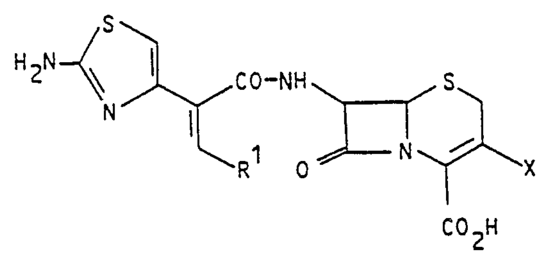

(I)

dans laquelle

$R^1$ a le sens indiqué à la revendication 6, et

X représente un reste usuel dans le cas des céphalosporines, procédé caractérisé en ce que :

a) on fait réagir des composés de formule IX

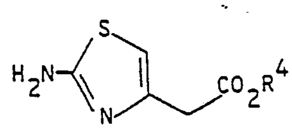

(IX)

avec un ester d'acide pyrocarbonique de formule

$$R^3—O—CO—O—CO—O—R^3$$

$R^3$ et $R^4$ étant tels que définis à la revendication 4,

b) on fait réagir le produit X ainsi obtenu

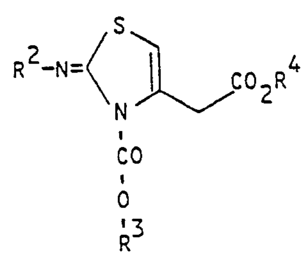

(X)

dans lequel $R^2$ représente $CO_2R^3$, et $R^3$ a le sens précité, tout d'abord avec une base convenable puis avec un aldéhyde de formule $R^1$—CHO pour obtenir le composé XI

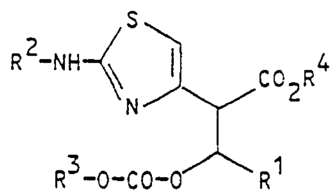

(XI)

$R^1$ ayant le sens précité ;

c) on fait réagir le composé XI avec une base pour obtenir le composé XII

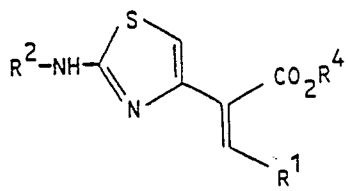

(XII)

d) on récupère à partir de XII, par séparation des isomères Z et E, puis saponification ou par saponification sélective, l'acide Z XIII

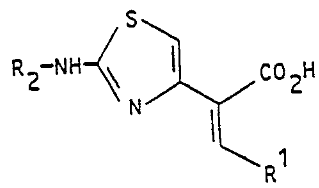

(XIII)

e) on fait réagir XIII avec un composé $Z—SO_2R^5$ pour obtenir un composé XVI

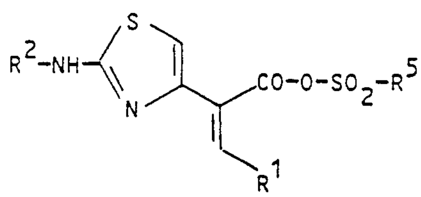

(XVI)

$R^5$ et Z ayant le sens indiqué à la revendication X ;

f) on copule XVI avec un acide céphalosporanique de formule XVII

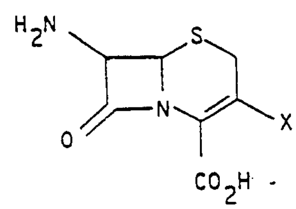

X ayant le sens précité, et

g) on élimine le groupe protecteur $R^2$.